(19) **Europäisches Patentamt**
**European Patent Office**
**Office européen des brevets**

(11) **EP 4 475 759 B1**

(12) **FASCICULE DE BREVET EUROPEEN**

(45) Date de publication et mention
de la délivrance du brevet:
**18.03.2026 Bulletin 2026/12**

(21) Numéro de dépôt: **23702826.1**

(22) Date de dépôt: **06.02.2023**

(51) Classification Internationale des Brevets (IPC):
***A61B 5/12*** *(2006.01)*

(52) Classification Coopérative des Brevets (CPC):
**A61B 5/123**

(86) Numéro de dépôt international:
**PCT/EP2023/052761**

(87) Numéro de publication internationale:
**WO 2023/152068 (17.08.2023 Gazette 2023/33)**

(54) **PROCÉDÉ DE DÉTERMINATION D'UNE INTENSITÉ DE MASQUAGE DANS UNE OREILLE CONTROLATÉRALE ET DISPOSITIF ÉLECTRONIQUE ASSOCIÉ**

VERFAHREN ZUR BESTIMMUNG EINER MASKIERUNGSINTENSITÄT IN EINEM KONTRALATERALEN OHR UND ZUGEHÖRIGE ELEKTRONISCHE VORRICHTUNG

METHOD FOR DETERMINING A MASKING INTENSITY IN A CONTRALATERAL EAR AND ASSOCIATED ELECTRONIC DEVICE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats de validation désignés:
**MA TN**

(30) Priorité: **08.02.2022 FR 2201114**

(43) Date de publication de la demande:
**18.12.2024 Bulletin 2024/51**

(73) Titulaire: **My Medical Assistant**
**51100 Reims (FR)**

(72) Inventeur: **WALLAERT, Nicolas**
**51100 Reims (FR)**

(74) Mandataire: **Radzimski, Eric**
**Cabinet Aubenard**
**21, boulevard Carnot**
**21000 Dijon (FR)**

(56) Documents cités:
**JP-A- S56 139 740      US-A1- 2004 097 826**

- **MARKLE O M ET AL: "The audiometer weber test as a means of determining the need for, and type of, masking", EMBASE, ELSEVIER SCIENCE PUBLISHERS, AMSTERDAM, NL, 1 January 1952 (1952-01-01), XP002807901**
- **WHITTLE ET AL: "A determination of the normal threshold of hearing by bone conduction", JOURNAL OF SOUND AND VIBRATION, ELSEVIER, AMSTERDAM , NL, vol. 2, no. 3, 1 July 1965 (1965-07-01), pages 227 - 248, XP024211538, ISSN: 0022-460X, [retrieved on 19650701], DOI: 10.1016/0022-460X(65)90110-0**
- **DOBIE R A ET AL: "Binaural interaction in auditory brain-stem responses: effects of masking", ELECTROENCEPHALOGRAPHY AND CLINICAL NEUROPHYSIOLOGY/EVOKED POTENTIALS SECTION, ELSEVIER, vol. 62, no. 1, 1 January 1985 (1985-01-01), pages 56 - 64, XP024105422, ISSN: 0168-5597, [retrieved on 19850101], DOI: 10.1016/0168-5597(85)90035-8**
- **DATABASE EMBASE [online] ELSEVIER SCIENCE PUBLISHERS, AMSTERDAM, NL; 1952, MARKLE O M ET AL: "The audiometer weber test as a means of determining the need for, and type of, masking", XP002807901, Database accession no. EMB-0008697050**

EP 4 475 759 B1

- **MARKLE O M ET AL: "The audiometer weber test as a means of determining the need for, and type of, masking", ANN. OTOL., ETC. 1952, vol. 61, no. 3, 1952, pages 888 - 900**

**Description**

L'invention concerne l'audiométrie tonale.

Des méthodes de test audiométriques sont connues de US-2004/097826-A1, JP-S56139740-A, WHITTLE ET AL: "A determination of the normal threshold of hearing by bone conduction", JOURNAL OF SOUND AND VIBRATION, vol. 2, no. 3, juillet 1965, ISSN: 0022-460X, DOI: 10.1016/0022-460X(65)90110-0, DOBIE R A ET AL: "Binaural interaction in auditory brain-stem responses: effects of masking", ELECTROENCEPHALOGRAPHY AND CLINICAL NEUROPHYSIOLOGY/EVOKED POTENTIALS SECTION, vol. 62, no. 1, janvier 1985, ISSN: 0168-5597, DOI: 10.1016/0168-5597(85)90035-8, et MARKLE O M ET AL: "The audiometer weber test as a means of determining the need for, and type of, masking", ANN. OTOL., ETC. 1952, vol. 61, no. 3, 1952, pages 888-900.

Les procédés de tests audiométriques permettent de mesurer de façon discrète, par voie osseuse et par voie aérienne, des seuils d'audition pour former un audiogramme sur une plage sonore s'étendant par exemple, de 125 à 8000 Hertz (de manière habituelle, les Hertz sont notés Hz dans la suite) en transmission aérienne et de 250 à 6000Hz en transmission osseuse.

Une série de sons de test est appliquée à l'oreille testée par exemple par l'intermédiaire d'un casque audio en conduction aérienne ou d'un vibrateur en conduction osseuse. Il est demandé au patient d'appuyer sur le bouton réponse dès lors qu'il entend un son de test.

Le test audiométrique peut être faussé si le son de test est perçu par l'oreille controlatérale (c'est-à-dire, l'oreille non testée). C'est pourquoi, le son de test doit être masqué par un son de masquage appliqué à l'oreille controlatérale en conduction aérienne, généralement par l'intermédiaire d'un casque audio. Toutefois, le son de masquage ne doit pas empêcher le son test d'être perçu par l'oreille testée. L'estimation du son de masquage répondant à ces contraintes est réalisée à partir du Rinne (à savoir la différence (en dB) entre la conduction aérienne et la conduction osseuse) et (des propriétés) du transfert transcrânien (transmission du son test à l'oreille controlatérale). Pour estimer le Rinne, dans l'état de l'art, on réalise préalablement un audiogramme en conduction aérienne et un audiogramme en conduction osseuse sans masquage, avant de réaliser les mêmes examens en masquant l'oreille controlatérale à l'aide du Rinne ainsi obtenu. La réalisation de ces audiogrammes allonge la durée du test audiométrique tout en fournissant une estimation imprécise du Rinne puisqu'aucun masquage n'est réalisé durant la réalisation de ces audiogrammes.

Pour remédier à ces inconvénients, l'invention concerne un procédé de test audiométrique comprenant les étapes suivantes :

- Application simultanée (S50) d'un son de test à une oreille testée (210) d'un patient comportant une intensité de test, et d'un son de masquage, comportant une intensité de masquage, à une oreille controlatérale (220) pour masquer le son de test,

le procédé étant caractérisé en ce qu'il comporte au moins l'un des deux couples d'étapes suivantes :

- Le premier couple d'étapes suivantes :

  - Détermination d'un Rinne théorique à partir d'un transfert transcrânien d'hypothèse,

- Détermination de l'intensité de masquage à partir du Rinne théorique.
- Le deuxième couple d'étapes suivantes :

  - Détermination d'un transfert transcrânien théorique à partir d'un Rinne d'hypothèse,
  - Détermination de l'intensité de masquage à partir du transfert transcrânien théorique.

Par exemple, en conduction aérienne, le transfert transcrânien d'hypothèse est compris entre 40dB et 80dB, par exemple, il est égal à 50 dB (de manière habituelle, et dans la suite, les décibels sont notés ici dB). En conduction osseuse, le transfert transcrânien d'hypothèse est compris entre -10dB et 20dB. L'origine de ces valeurs est donnée à l'annexe 1 et 2.

Par exemple le Rinne d'hypothèse est compris entre 0 et 70 dB.

Ainsi, le masquage est déterminé sans nécessiter de réaliser une audiométrie en conduction osseuse et en conduction aérienne préalable. L'invention permet ainsi de rendre plus rapide les diagnostics audiométriques d'un patient. L'intensité de masquage est ainsi déterminée à partir d'un Rinne théorique obtenu à partir d'un transfert transcrânien d'hypothèse (i.e. : d'une hypothèse sur un transfert transcrânien) ou est déterminé à partir d'un transfert transcrânien théorique obtenu à partir d'un Rine d'hypothèse (i.e. : d'une hypothèse sur le Rinne). En variante, l'intensité de masquage est déterminée en testant plusieurs valeurs de Rinne ou de transfert trans cranien.

Dans un mode de réalisation, le procédé comporte l'une au moins des deux doublets d'étapes (donc éventuellement, les deux doublets en même temps) suivants, le patient (200) comportant un transfert transcrânien :

- Premier doublet d'étapes :

  - Répétition des (deux) étapes suivantes :

    - Détermination d'un Rinne courant,
    - Détermination (S30) que le Rinne courant est compatible, s'il existe une intensité courante, telle que si l'intensité de

    masquage est égale à l'intensité courante et le Rinne de l'oreille de test (210) ou de l'oreille controlatérale (220) est égale au Rinne courant, alors le son de test n'est pas perçu par l'oreille controlatérale (220) et le son de masquage n'empêche pas la perception du son de test par l'oreille testée (210),

  - Détermination d'une intensité de masquage à partir d'un Rinne courant compatible maximal déterminé durant l'étape de répétition,

- Deuxième doublet d'étapes :

  - Répétition des (deux) étapes suivantes :

    - Détermination d'un transfert transcrânien courant,
    - Détermination (S30) que le transfert transcrânien courant est compatible, s'il existe une intensité courante, telle que si l'intensité de masquage est égale à l'intensité courante et le transfert transcrânien du patient (200) est égale au transfert transcrânien courant, alors le son de test n'est pas perçu par l'oreille controlatérale (220) et le son de masquage n'empêche pas la perception du son de test par l'oreille testée (210),

  - Détermination d'une intensité de masquage à partir d'un transfert transcrânien courant compatible maximal déterminé durant l'étape de répétition.

Par répétition, on entend que les (deux) étapes ci-dessus peuvent être réalisées deux fois ou plus (pour le même son de test).

Dans un mode de réalisation, les (deux) étapes sont répétées, pour le premier doublet d'étapes, jusqu'à ce que le Rinne courant soit compatible, ou pour le deuxième doublet d'étapes, jusqu'à ce que le transfert transcrânien soit compatible.

Selon un mode de réalisation, l'étape de détermination d'un Rinne courant comporte une étape de décrémentation du Rinne courant d'un pas, le procédé comprenant en outre une étape d'initialisation du Rinne courant à un Rinne d'initialisation.

En variante, des valeurs du Rinne courant peuvent être balayées de manières différentes (selon un Rinne courant croissant par exemple).

Selon un mode de réalisation, le Rinne d'initialisation est compris entre 55 dB et 65 dB, préférentiellement égal à 60 dB. Ce Rinne d'initialisation est supérieur au Rinne réel de la plupart des patients.

Selon un mode de réalisation, le pas est compris entre 1 dB et 20 dB, préférentiellement égal à 5 dB.

Selon un exemple de réalisation, si le Rinne courant compatible maximal est inférieur à un seuil minimum, un message d'alarme est envoyé (et affiché).

Le seuil minimum est par exemple compris entre 0 dB et 45 dB, par exemple égal à 40 dB. Cette valeur de seuil minimal est déterminée à partir des résultats d'un test de Weber préalablement obtenu. Par exemple, le procédé de test comprend un test de Weber et si le test de Weber se latéralise du côté de l'oreille la meilleure, nous sommes face à une probable surdité de perception (ie. Venant de l'oreille interne). Le seuil minimum peut par exemple être compris entre 0 et 30 dB, et par exemple être égal à 20dB, car le Rinne ne doit à priori pas être conséquent. A l'inverse, si le test de Weber se latéralise du côté de l'oreille la mauvaise oreille, nous sommes face à une probable surdité de transmission ou mixte (ie : affection de l'oreille externe ou moyenne). Le seuil minimum peut par exemple dans ce cas compris entre 25 et 65 dB et être fixé à 40dB car il est alors improbable d'avoir un Rinne assez faible.

Ainsi, le test audiométrique selon l'invention peut comprendre un interrogatoire du patient pour connaitre sa meilleure oreille et un test de Weber, et dans lequel le seuil minimum est compris entre 0 et 40 dB si le test de Weber se latéralise du côté de l'oreille la meilleure et entre 25 et 65 si le test de Weber se latéralise du côté opposé à l'oreille la meilleure. Pour certains patients, le Rinne réel peut en effet être inférieur au seuil minimum et éventuellement être nul (i.e. égal à 0 dB).

Par exemple, l'étape de détermination que le Rinne courant (ou le Rinne théorique) est compatible comprend les étapes suivantes :

- Calcul à partir de l'intensité de test (et du Rinne courant) d'un seuil d'efficacité pour l'intensité de masquage au-dessus duquel le son de test ne peut pas être perçu par l'oreille controlatérale,
- Calcul à partir de l'intensité de test (et du Rinne courant) d'un seuil de retentissement pour l'intensité de masquage au-dessous duquel le son de masquage n'empêche pas la perception du son de test (autrement dit : au-dessous duquel le son de test est perçu en présence du son de masquage) par l'oreille testée,
- Une étape de comparaison du seuil d'efficacité et du seuil de retentissement,

le Rinne courant (ou le Rinne théorique) étant compatible si le seuil d'efficacité est inférieur ou égal au seuil de retentissement.

Par exemple, le test audiométrique étant réalisé en conduction aérienne (i.e. : le son de test est appliqué à l'oreille testée en conduction aérienne, par exemple à l'aide d'un casque audio, et le son de masquage est appliqué à l'oreille controlatérale en conduction aérienne, par exemple à l'aide d'un casque audio), et le calcul du seuil de retentissement est réalisé à partir du Rinne courant.

Par exemple, en conduction aérienne, le seuil de retentissement est égal à :

-

$$SR = IT - \text{Rinne courant}_{\text{oreille testée}} + TTC - RSBR,$$

où

- $SR$ est le seuil de retentissement (en dB),
- $IT$ est l'intensité de test (en dB),
- $TTC$ est le transfert transcrânien (en dB), (autrement dit : une hypothèse sur le transfert transcrânien, car celui-ci ne peut être mesuré sur le patient)
- Rinne courant $_{\text{oreille testée}}$ est le Rinne courant de l'oreille testée (210).
- $RSBR$ est le rapport signal sur bruit (pour que le son de masquage appliqué à l'oreille controlatérale n'empêche pas le son de test d'être entendu dans l'oreille de test).

Par exemple, en conduction osseuse (i.e. : le son test est appliqué à l'oreille testée en conduction osseuse, par exemple à l'aide d'un vibrateur, et le son de masquage est appliqué à l'oreille controlatérale en conduction aérienne, par exemple à l'aide d'un casque), le seuil de retentissement est égal à :

-

$$SR = IT + TTC - RSBR,$$

avec les mêmes notations que ci-dessus.

Par exemple, le rapport signal sur bruit RSBR est inférieur à 10 dB, par exemple égal à 0 dB.

Selon un mode de réalisation, le calcul du seuil d'efficacité est réalisé à partir du Rinne courant.

Par exemple, en conduction aérienne, le seuil d'efficacité est égal à :

-

$$SE = IT - TTC + RSBE + \text{Rinne courant}_{\text{contro}},$$

avec les mêmes notations que ci-dessus, et où

- $SE$ est le seuil d'efficacité (en dB),
- Rinne courant $_{\text{contro}}$ est le Rinne courant de l'oreille contralérale,
- $RSBE$ est le rapport signal sur bruit (pour que le son de masquage couvre le son de test dans l'oreille controlatérale).

Par exemple, en conduction osseuse, le seuil d'efficacité est égal à :

-

$$SE = IT + RSBE + Rinne\ courant\ _{contro},$$

avec les mêmes notations que ci-dessus.

Par exemple, le rapport signal sur bruit RSBE est supérieur à 15 dB, par exemple égale à (+)20 dB.
Par exemple, en conduction aérienne, le transfert transcrânien est compris entre 40dB et 80dB, par exemple, il est égal à 50 dB. En conduction osseuse, le transfert transcrânien est compris entre -10dB et 20dB. La valeur du transfert transcrânien peut être ajustée en fonction de la fréquence, tant en conduction aérienne qu'en conduction osseuse.
Selon un mode de réalisation, le Rinne de l'oreille controlatérale peut prendre (autrement dit : être remplacé par) une valeur fixe égale à :

- Une valeur fonction du seuil d'un audiogramme en conduction aérienne de l'oreille controlatérale si celui-ci est (préalablement) connu (pour la fréquence du son de test, et ce test audiométrique précédent peut faire partie du procédé selon l'invention), par exemple, le seuil de l'audiogramme divisé par une valeur supérieure à 2, par exemple, par 3 par exemple, si le test de Weber se latéralise du côté de la meilleure oreille, ou en variante par une valeur comprise entre 1 et 4, par exemple égale à 1,5 si le weber se latéralisé du côté de l'oreille la moins bonne, ou
- Une valeur de Rinne de l'oreille controlatérale estimée à partir d'un test audiométrique précédent (et ce test audiométrique précédent peut faire partie du procédé selon l'invention) (par exemple, si le choix des sons de test est réalisé par un processus gaussien, la valeur du Rinne de l'oreille controlatérale peut être obtenue à partir de l'initialisation de ce processus gaussien qui peut être réalisée pour les deux oreilles en conduction osseuse et en conduction aérienne, avant de débuter le choix de sons de tests par le processus gaussien pour les deux oreilles en conduction osseuse et en conduction aérienne ou à partir de l'estimation courante du Rinne lorsque le processus gaussien est enclenché).

L'étape de répétition n'est pas réalisée sur le Rinne de l'oreille controlatérale dans ce cas (mais sur le Rinne de l'oreille en cours de test).
Selon un mode de réalisation, le Rinne (par exemple théorique) ne peut être supérieur à un seuil maximal, par exemple à 60 dB. Par exemple, si la valeur fonction de l'audiogramme en conduction aérienne est supérieure au seuil maximal, le Rinne (par exemple théorique) est ramené au seuil haut.
Selon un mode de réalisation, durant l'étape de détermination de l'intensité de masquage, le Rinne courant prenant comme valeur le Rinne courant compatible maximum :

- Si le seuil d'efficacité et le seuil de retentissement sont égaux, alors l'intensité de masquage prend comme valeur le seuil d'efficacité, où
- Si le seuil d'efficacité est inférieur au seuil de retentissement, alors l'intensité de masquage prend comme valeur une valeur intermédiaire entre les seuils d'efficacité et de retentissement (compris), par exemple la moyenne arithmétique du seuil de retentissement et du seuil d'efficacité.

Selon une variante, le Rinne théorique peut être déterminé en conduction osseuse, en résolvant l'équation :

IT + RSBE + Rinne théorique = IT + TTC- RSBR. (Soit Rinne théorique = TTC - RSBE - RSBR).

Par construction, ce Rinne théorique est compatible, bien entendu.
Selon une variante, de la même manière, un transfert transcrânien théorique peut être déterminé en conduction osseuse, en résolvant la même équation, à partir d'un Rinne d'hypothèse (autrement dit : d'une hypothèse sur le Rinne théorique).
L'intensité de masquage prend alors par exemple comme valeur le seuil d'efficacité SE, telle que défini plus haut.
Selon une variante, l'étape de détermination que le Rinne courant est compatible comprend les étapes suivantes :

- Calcul d'une intensité suffisante pour masquer le son de test dans l'oreille controlatérale à partir de l'intensité de test (et éventuellement du Rinne courant),

- Calcul du retentissement du son de masquage dans l'oreille à tester à partir de l'intensité suffisante (et éventuellement du Rinne courant),

le Rinne courant étant compatible si le retentissement du son de masquage est inférieur à l'intensité de test plus un rapport signal sur bruit, par exemple égale à 0 dB.

Par exemple, l'intensité de masquage est plafonnée à un seuil haut, par exemple égale à 85 dB. Par exemple, si la valeur fonction de l'audiogramme en conduction aérienne est supérieure au seuil, le Rinne est ramené au seuil haut.

Au lieu de déterminer un Rinne théorique à partir d'une hypothèse sur le transfert transcrânien, on peut déterminer un transfert transcrânien théorique à partir d'une hypothèse sur le Rinne sans sortir du cadre de l'invention, d'une manière analogue à ce qui est décrit dans cette demande pour déterminer le Rinne théorique. Les caractéristiques selon l'invention pour le cas où on détermine un transfert transcrânien théorique à partir d'une hypothèse sur le Rinne sont analogues au cas où on détermine un Rinne théorique à partir d'une hypothèse sur le transfert transcrânien, C'est pourquoi on ne détaille pas ici ces caractéristiques.

Selon un mode de réalisation :

- Le test audiométrique est réalisé en conduction aérienne, et l'oreille testée (en premier) est la meilleur oreille (comportant une oreille opposée), le procédé de test comprenant ensuite un test audiométrique de l'oreille opposée de l'oreille testée en conduction aérienne (selon l'invention), où
- Le test audiométrique est réalisé en conduction osseuse, et l'oreille testée est l'oreille où s'est latéralisé un test de Weber (le procédé selon l'invention peut comprendre par exemple le test de Weber), le procédé de test comprenant ensuite un test audiométrique de l'oreille opposée à l'oreille testée en conduction osseuse (selon l'invention).

Ainsi, on débute par l'oreille la meilleure en conduction aérienne, et par l'oreille qui a probablement le plus grand Rinne en conduction osseuse (l'oreille où s'est latéralisé le test de Weber).

Le procédé selon l'invention peut être réalisé (autrement dit : mis en œuvre) par un dispositif électronique de test audiométrique. Le dispositif électronique peut comprendre une unité centrale électronique (par exemple comprise dans un téléphone mobile ou une tablette tactile électronique) et un casque audio ou des inserts, ou des haut-parleurs pour appliquer les sons à l'oreille et le masquage de l'oreille controlatérale en transmission aérienne. En transmission osseuse, on utilise un ou plusieurs vibrateurs. L'acquisition de l'information selon laquelle un son est entendu ou pas peut-être réalisée par un bouton sur lequel le patient appuie lorsqu'il entend un son, ou par commande vocale ou par détection d'image.

L'invention concerne donc aussi un dispositif électronique de test audiométrique configuré pour mettre en œuvre les étapes du procédé selon l'invention.

L'invention concerne en outre un programme d'ordinateur comprenant des instructions, exécutables par un microprocesseur ou un microcontrôleur, pour la mise en œuvre du procédé selon l'invention.

Les caractéristiques et avantages du dispositif électronique et du programme d'ordinateur sont identiques à ceux du procédé, c'est pourquoi, ils ne sont pas repris ici.

On entend qu'un élément tel que le dispositif électronique de test audiométrique, l'unité centrale, ou un autre élément est « configuré pour » réaliser une étape ou une opération, par le fait que l'élément comporte des moyens pour (autrement dit « est conformé pour » ou « est adapté pour ») réaliser l'étape ou l'opération. Il s'agit préférentiellement de moyens électroniques, par exemple un programme d'ordinateur, des données en mémoire et/ou des circuits électroniques spécialisés.

Lorsqu'une étape ou une opération est réalisée par un tel élément, cela implique généralement que l'élément comporte des moyens pour (autrement dit « est conformé pour » ou « est adapté pour ») réaliser l'étape ou l'opération. Il s'agit également par exemple de moyens électroniques, par exemple un programme d'ordinateur, des données en mémoire et/ou des circuits électroniques spécialisés.

D'autres caractéristiques et avantages de la présente invention apparaitront plus clairement à la lecture de la description détaillée qui suit comprenant des modes de réalisation de l'invention donnés à titre d'exemples nullement limitatifs et illustrés par les dessins annexés, dans lesquels :

[Fig.1] représente un dispositif électronique selon un mode de réalisation de l'invention.

[Fig.2] représente le procédé selon l'invention, dans un exemple de réalisation, mis en œuvre par le dispositif électronique de la [Fig.1].

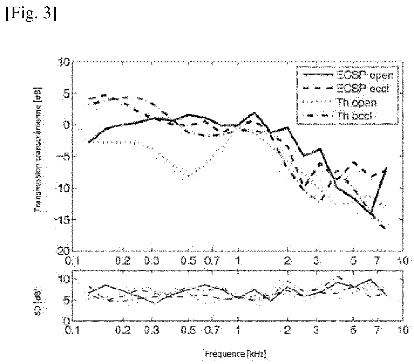

[Fig.3] Estimations moyennes du transfert transcranien obtenus par des mesures ECSP pour des conduits auditifs ouverts (ligne pleine) et occlus (ligne pointillée), et obtenues par des seuils auditifs BC (Bone Conducted, ou conduction osseuse) pour des conduits auditifs ouverts, nommé Th ouverts (ligne pointillée) et occlus, nommé Th occlus (ligne pointillée). Les résultats des écarts types, nommés SD, utilise les mêmes symboles pour la légende.

**Description détaillée d'un exemple de réalisation de l'invention**

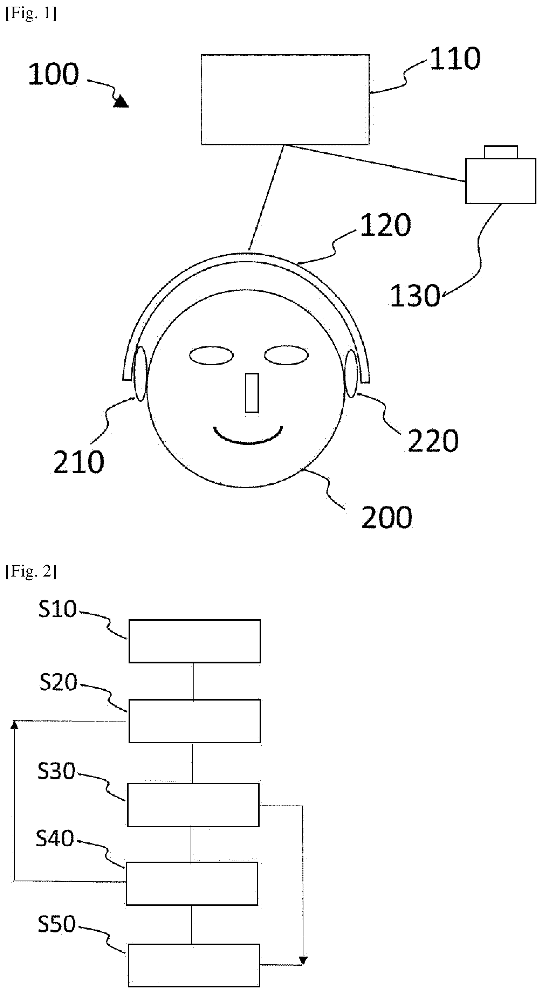

En référence aux figures 1 et 2, pour un test audiométrique en conduction osseuse, afin de déterminer l'intensité de masquage pour une intensité de test, à l'étape S10, l'unité centrale 110 initialise en mémoire un Rinne courant $_{contro}$ à 60 dB, et à l'étape S20, l'unité centrale 110 calcule SE et SR tel que :

-

$$SR = IT + 50,$$

et

-

$$SE = IT + 20 + \text{Rinne courant }_{contro},$$

où
- SE est le seuil d'efficacité (en dB), SR est le seuil de retentissement (en dB),
- IT est l'intensité de test (en dB),
- Rinne courant $_{contro}$ est le Rinne courant de l'oreille contralérale.

On fait ainsi ici l'hypothèse d'un transfert trans-crânien de 50 dB.

A l'étape S30, SE et SR sont comparés par l'unité centrale 110, si SR=SE alors le Rinne$_{contro}$ est le Rinne courant compatible maximal et l'intensité de masquage est égale à SR (et SE). Si SR=SE ou SE<SR, alors le Rinne$_{contro}$ est le Rinne courant compatible maximal, et l'intensité de masquage est égale à la moyenne arithmétique du seuil de masquage et du seuil d'efficacité. Toutefois, si l'intensité de masquage ainsi déterminée est supérieure à 85 dB, elle est ramenée à 85 dB. Si SR=SE ou SE<SR, alors le procédé se poursuit à l'étape S50. A l'étape S40, si SE>SR, alors le Rinne courant $_{contro}$ est décrémenté de 5 dB, et le procédé se poursuit à l'étape S20.

A l'étape S50, l'unité centrale commande l'émission d'un son de test dans l'oreille testé 210, le son ayant une intensité de test, et du son de masquage dans l'oreille controlatérale 220 par l'intermédiaire du casque 120. Si le Rinne courant$_{contro}$ (c'est-à-dire le Rinne compatible maximal) est inférieur à 40 dB, alors un message d'alerte est transmis à un écran (non représenté pour affichage). Le message d'alerte comporte par exemple le texte suivant : « Audiométrie incertaine - Réalisation d'une audiométrie avec masquage ipsilatéral de Rainville recommandée pour confirmer ».

En conduction aérienne, on peut réaliser les étapes S10 à S50, mais avec :

-

$$SR = IT - \text{Rinne courant }_{\text{oreille testée}} + 50,$$

et

-

$$SE = IT - 50 + 20 + \text{Rinne courant }_{contro},$$

où
- SE est le seuil d'efficacité (en dB), SR est le seuil de retentissement (en dB),
- IT est l'intensité de test (en dB),
- Rinne courant $_{contro}$ est le Rinne courant de l'oreille controlalérale,
- Rinne courant $_{\text{oreille testée}}$ est le Rinne courant de l'oreille testée.

Le Rinne courant$_{\text{oreilletestée}}$ peut être initialisé à l'étape S10 et décrémenté à l'étape S30, simultanément au Rinne courant$_{contro}$,, dans une boucle imbriquée avec celle où le Rinne courant$_{contro}$ est décrémenté ou dans une boucle différente.

Rinne courant$_{contro}$ peut être remplacé par l'une des valeurs fixes suivantes :

- Une valeur fonction du seuil d'un audiogramme en conduction aérienne de l'oreille controlatérale si celui-ci est (préalablement) connu (pour la fréquence du son de test, et ce test audiométrique précédent peut faire partie du procédé selon l'invention), par exemple, le seuil de l'audiogramme divisé par 3 (par exemple, si le test de Weber se latéralise du côté de la meilleure oreille) ou 1,5 (si le weber se latéralisé du côté de l'oreille la moins bonne), ou

- Une valeur de Rinne de l'oreille controlatérale estimée à partir d'un test audiométrique précédent (et ce test audiométrique précédent peut faire partie du procédé selon l'invention) (par exemple, si le choix des sons de test est réalisé par un processus gaussien, la valeur du Rinne de l'oreille controlatérale peut être obtenue à partir de l'initialisation de ce processus gaussien qui peut être réalisée pour les deux oreilles en conduction osseuse et en conduction aérienne, avant de débuter le choix de sons de tests par le processus gaussien pour les deux oreilles en conduction osseuse et en conduction aérienne ou à partir du Rinne issu de l'estimation de seuil du processus gaussien).

Les étapes d'initialisation et de décrémentation du Rinne de l'oreille controlatérale ne sont pas réalisées dans ce cas (mais celle de Rinne de l'oreille à tester le sont).

En variante, l'unité centrale peut déterminer une intensité de masquage, par exemple à partir d'un son de test de 10 dB en conduction osseuse de la manière suivante :

Le son de test arrive dans l'oreille controlatérale à 10 dB. L'unité centrale détermine que 90 dB sont suffisants pour masquer (en transmission aérienne) dans l'oreille controlatérale, avec une hypothèse de Rinne controlatérale à 60 dB (le rapport signal sur bruit pour que le son de masquage masque le son de test étant fixé à 20 dB). Avec un transfert transcrânien de 50 dB, l'oreille testée reçoit 40 dB du son de masquage, ce qui empêche le son de test d'être entendu dans l'oreille testée (le rapport signal sur bruit est égal à -30 dB).

Avec une hypothèse de Rinne controlatérale à 40 dB, 70 dB sont suffisant pour masquer le son de test dans l'oreille controlatérale. Avec un transfert transcrânien de 50 dB, l'oreille testée reçoit 20 dB du son de masquage, ce qui empêche le son de test d'être entendu dans l'oreille testée (le rapport signal sur bruit est égal à -10 dB).

Avec une hypothèse de Rinne controlatérale à 30 dB, 60 dB sont suffisant pour masquer le son de test dans l'oreille controlatérale. Avec un transfert transcrânien de 50 dB, l'oreille testée reçoit 10 dB du son de masquage, ce qui n'empêche pas le son de test d'être entendu dans l'oreille testée (le rapport signal sur bruit est égal à 0 dB). Il faut donc masquer à une intensité 60 dB.

Si le weber était latéralisé du côté de la bonne oreille, le Rinne courant$_{contro}$ (c'est-à-dire le Rinne compatible maximal) est supérieur à la valeur seuil de Rinne (e.g. 20dB), et aucun message d'alerte ne s'affiche. Si toutefois le weber était latéralisé du côté de l'oreille la plus sourde, le Rinne courant$_{contro}$ (c'est-à-dire le Rinne compatible maximal) est inférieur à 40 dB, donc un message d'alerte est transmis à un écran (non représenté pour affichage). Le message d'alerte comporte par exemple le texte suivant : « Audiométrie incertaine - Réalisation d'une audiométrie avec masquage ipsilatéral de Rainville recommandée pour confirmer ».

Selon un mode de réalisation, le test audiométrique pour un patient est réalisé dans l'ordre chronologique suivant :

- Interrogatoire du patient pour connaitre sa meilleure oreille
- Test de Weber : Stimulation binaurale ou frontale en conduction osseuse
- On note vers quelle oreille est latéralisé le son,
- Test en conduction aérienne de la bonne oreille,
- Test en conduction aérienne de l'oreille opposée,
- Test en conduction osseuse de l'oreille vers laquelle est latéralisée le son,
- Test en conduction osseuse de l'oreille opposée.

L'interrogatoire du patient permet de déterminer ce qui est a priori sa meilleure oreille

Par exemple, durant le procédé de test selon l'invention, un processus gaussien peut-être mis en œuvre par l'unité centrale 110 pour déterminer les sons à tester, par exemple comme décrit dans l'article de Schlittenlacher J, Turner RE, Moore BCJ. « Audiogram estimation using Bayesian active learning » (J Acoust Soc Am. 2018;144(1):421).

Le procédé selon l'invention peut être réalisé (autrement dit : mise en œuvre) par un dispositif électronique 100 de test audiométrique. Le dispositif électronique 100 peut comprendre une unité centrale 110 et un casque audio 120 pour appliquer les sons à l'oreille 210 et le masquage de l'oreille controlatérale 220 en transmission aérienne d'un patient 200. En transmission osseuse, on utilise un ou plusieurs vibrateurs (non représenté) pour l'oreille testée 210 en combinaison avec un casque audio en transmission aérienne pour le masquage de l'oreille controlatérale 220. L'acquisition de l'information selon laquelle un son est entendu ou pas peut-être réalisée par un bouton 130 sur lequel le patient appuie lorsqu'il entend un son.

ANNEXE 1 : Origine des valeurs de transfert transcrânien en CA.

Les tableaux suivants présentent les atténuations interaurale minimale (transferts transcrânien minimum observable) pour le casque THD 39 (aérien) et les inserts audiométriques (aérien)

• Katz, J., Lezynski, J. (2002). Clinical Masking. Dans J. Katz, ed., Handbook of Clinical Audiology, Williams and Wilkins, Baltimore.

• Munro, K.J., Agnew, N. A comparison of inter-aural attenuation with the Etymotic ER-3A insert earphone and the Telephonics TDH-39 supra-aural earphone. Br J Audiol 1999 ; 33 : 259-262.
• Hall, JW., MUELLER, HG. (1997). The audiologists' desk reference, Volume I, Singular Publishing Group, San DiegoMin IA (casque supra-auriculaire : TDH-39), spécifique pour chacune des fréquences audiométriques

| Hz | dB | Référence bibliographique |
|---|---|---|
| 125 | 35 | Katz & Lezynski, (2002) |
| 250 | 48 | Munro & Agnew, BJA (1999) |
| 500 | 44 | Munro & Agnew, BJA (1999) |
| 750 | 40 | N/A - fulfill traditional approach |
| 1000 | 48 | Munro & Agnew, BJA (1999) |
| 1500 | 40 | N/A - fulfill traditional approach |
| 2000 | 44 | Munro & Agnew, BJA (1999) |
| 3000 | 56 | Hall J.W. III & Mueller G.H. III / Munro & Agnew, BJA (1999) |
| 4000 | 50 | Katz J / Munro & Agnew, BJA (1999) |
| 6000 | 44 | Hall J.W. III & Mueller G.H. III / Munro & Agnew, BJA (1999) |
| 8000 | 42 | Katz J / Munro & Agnew, BJA (1999) |

Min IA (inserts audiométriques), spécifique pour chacune des fréquences audiométriques

| Hz | dB | Référence bibliographique |
|---|---|---|
| 125 | 60 | N/A - traditional value |
| 250 | 72 | Munro & Agnew, BJA (1999) |
| 500 | 64 | Munro & Agnew, BJA (1999) |
| 750 | 60 | N/A - traditional value |
| 1000 | 58 | Munro & Agnew, BJA (1999) |
| 1500 | 60 | N/A - traditional value |
| 2000 | 56 | Munro & Agnew, BJA (1999) |
| 3000 | 58 | Munro & Agnew, BJA (1999) |
| 4000 | 72 | Munro & Agnew, BJA (1999) |
| 6000 | 54 | Munro & Agnew, BJA (1999) |
| 8000 | 62 | Munro & Agnew, BJA (1999) |

ANNEXE 2 : Origine des valeurs données en transfert trasncranien en CO

**Revendications**

1. Procédé de test audiométrique comprenant les étapes suivantes :

- Application simultanée (S50) d'un son de test à une oreille testée (210) d'un patient (200) comportant une intensité de test, et d'un son de masquage, comportant une intensité de masquage, à une oreille controlatérale (220) pour masquer le son de test,

le procédé étant **caractérisé en ce qu'**il comporte au moins l'un des deux couples d'étapes suivantes :

- Le premier couple d'étapes suivantes :

- Détermination d'un Rinne théorique à partir d'un transfert transcrânien,
- Détermination de l'intensité de masquage à partir du Rinne théorique

- Ou, le deuxième couple d'étapes suivantes :

- Détermination d'un transfert transcrânien théorique à partir d'un Rinne théorique,
- Détermination de l'intensité de masquage à partir du transfert transcrânien théorique.

2. Procédé de test audiométrique selon la revendication précédente comportant en outre l'une au moins des deux doublets d'étapes suivants, le patient (200) comportant un transfert transcrânien :

- Premier doublet d'étapes :

- Répétition des étapes suivantes :

- Détermination d'un Rinne courant,
- Détermination (S30) que le Rinne courant est compatible, s'il existe une intensité courante, telle que si l'intensité de masquage est égale à l'intensité courante et le Rinne de l'oreille de test (210) ou de l'oreille controlatérale (220) est égale au Rinne courant, alors le son de test n'est pas perçu par l'oreille controlatérale (220) et le son de masquage n'empêche pas la perception du son de test par l'oreille testée (210),

- Détermination d'une intensité de masquage à partir d'un Rinne courant compatible maximal déterminé durant l'étape de répétition,

- Deuxième doublet d'étapes :

- Répétition des étapes suivantes :

- Détermination d'un transfert transcrânien courant,
- Détermination (S30) que le transfert transcrânien courant est compatible, s'il existe une intensité courante, telle que si l'intensité de masquage est égale à l'intensité courante et le transfert transcrânien du patient (200) est égale au transfert transcrânien courant, alors le son de test n'est pas perçu par l'oreille controlatérale (220) et le son de masquage n'empêche pas la perception du son de test par l'oreille testée (210),

- Détermination d'une intensité de masquage à partir d'un transfert transcrânien courant compatible maximal déterminé durant l'étape de répétition.

3. Procédé de test audiométrique selon la revendication précédente, dans lequel le procédé de test audiométrique comprend le premier doublet d'étapes, et dans lequel l'étape de détermination d'un Rinne courant comporte une étape de décrémentation (S40) du Rinne courant d'un pas, le procédé comprenant en outre une étape d'initialisation (S10) du Rinne courant à un Rinne d'initialisation.

4. Procédé de test audiométrique selon l'une quelconque des revendications 2 ou 3 dans lequel le procédé de test audiométrique comprend le premier doublet d'étapes, et dans lequel si le Rinne courant compatible maximal est inférieur à un seuil minimum, un message d'alarme est envoyé.

5. Procédé de test audiométrique selon l'une quelconque des revendications 2 à 4 dans lequel le procédé de test audiométrique comprend le premier doublet d'étapes, et dans lequel l'étape de détermination que le Rinne courant est compatible comprend les étapes suivantes :

- Calcul d'un seuil d'efficacité pour l'intensité de masquage au-dessus duquel le son de test ne peut être perçu par l'oreille controlatérale (220), à partir de l'intensité de test,
- Calcul d'un seuil de retentissement pour l'intensité de masquage au-dessous duquel le son de masquage n'empêche pas la perception du son de test par l'oreille testée (210) à partir de l'intensité de test,
- Une étape de comparaison du seuil d'efficacité et du seuil de retentissement,

le Rinne courant étant compatible si le seuil d'efficacité est inférieur ou égal au seuil de retentissement.

6. Procédé de test audiométrique selon la revendication 2 à 5, le test audiométrique étant réalisé en conduction aérienne, dans lequel le procédé de test audiométrique comprend le premier doublet d'étapes, et dans lequel le calcul du seuil d'efficacité est réalisé à partir du Rinne courant.

7. Procédé de test audiométrique selon la revendication 2 ou 6, dans lequel le procédé de test audiométrique comprend le premier doublet d'étapes, et dans lequel le calcul seuil de retentissement est réalisé à partir du Rinne courant.

8. Procédé de test audiométrique selon l'une quelconque des revendications 5 à 7 dans lequel le procédé de test audiométrique comprend le premier doublet d'étapes, et dans lequel, durant l'étape de détermination de l'intensité de masquage, le Rinne courant prenant comme valeur le Rinne courant compatible maximum :

   - Si le seuil d'efficacité et le seuil de retentissement sont égaux, alors l'intensité de masquage prend comme valeur le seuil d'efficacité, ou
   - Si le seuil d'efficacité est inférieur au seuil de retentissement, alors l'intensité de masquage prend comme valeur une valeur intermédiaire entre le seuil d'efficacité et le seuil de retentissement.

9. Procédé de test audiométrique selon l'une quelconque des revendications 2 à 4 dans lequel le procédé de test audiométrique comprend le deuxième doublet d'étapes, et dans lequel l'étape de détermination que le Rinne courant est compatible comprend les étapes suivantes :

   - Calcul d'une intensité suffisante pour masquer le son de test dans l'oreille controlatérale (220) à partir de l'intensité de test,
   - Calcul du retentissement du son de masquage dans l'oreille testée (210) à partir de l'intensité suffisante,

   le Rinne courant étant compatible si le retentissement du son de masquage est inférieur à l'intensité de test plus un rapport signal sur bruit.

10. Procédé de test audiométrique selon l'une quelconque des revendications 1 à 9 dans lequel :

   - Le test audiométrique est réalisé en conduction aérienne, et l'oreille testée (210) est la meilleure oreille, le procédé de test comprenant ensuite un test audiométrique de l'oreille opposée (220) à l'oreille testée (210) en conduction aérienne, où
   - Le test audiométrique est réalisé en conduction osseuse, et l'oreille testée (210) est l'oreille où s'est latéralisé un test de Weber, le procédé de test comprenant ensuite un test audiométrique de l'oreille opposée (220) à l'oreille testée (210) en conduction osseuse.

11. Dispositif électronique (100) de test audiométrique configuré pour mettre en œuvre les étapes du procédé selon l'une quelconque des revendications 1 à 10.

12. Programme d'ordinateur comprenant des instructions, qui conduisent le dispositif selon la revendication 11 à exécuter les étapes du procédé selon l'une quelconque des revendications 1 à 10.

**Patentansprüche**

1. Audiometrisches Testverfahren umfassend die folgenden Schritte:

   - simultane Anwendung (S50) eines Testklangs an einem getesteten Ohr (210) eines Patienten (200) mit einer Testintensität und eines Maskierungsklangs mit einer Maskierungsintensität an einem kontralateralen Ohr (220), um den Testklang zu maskieren;

   **dadurch gekennzeichnet, dass** es mindestens eines der beiden Paare folgender Schritte umfasst:

   - das erste Paar folgender Schritte:

      ∘ Bestimmung eines theoretischen Rinne-Testergebnisses aus einer transkraniellen Übertragung,

◦ Bestimmung der Maskierungsintensität aus dem theoretischen Rinne-Testergebnis

- oder das zweite Paar folgender Schritte:

◦ Bestimmung einer theoretischen transkraniellen Übertragung aus dem theoretischen Rinne-Testergebnis,
◦ Bestimmung der Maskierungsintensität aus der theoretischen transkraniellen Übertragung.

2. Audiometrisches Testverfahren nach dem vorhergehenden Anspruch ferner umfassend mindestens eine der beiden Dubletten von folgenden Schritten, wobei der Patient (200) eine transkranielle Übertragung aufweist:

- erste Dublette von Schritten:

◦ Wiederholung der folgenden Schritte:

▪ Bestimmung eines aktuellen Rinne-Testergebnisses,
▪ Bestimmung (S30), dass das aktuelle Rinne-Testergebnis kompatibel ist, wenn eine aktuelle Intensität vorhanden ist, z. B., wenn die Maskierungsintensität gleich der aktuellen Intensität ist und das Rinne-Testergebnis des getesteten Ohrs (210) oder des kontralateralen Ohrs (220) gleich dem aktuellen Rinne-Testergebnis ist; dann wird der Testklang vom kontralateralen Ohr (220) nicht wahrgenommen, und der Maskierungsklang verhindert nicht, dass das getestete Ohr (210) den Testklang wahrnimmt,

◦ Bestimmung einer Maskierungsintensität aus einem während des Wiederholungsschritts bestimmten maximalen kompatiblen aktuellen Rinne-Testergebnis;

- zweite Dublette von Schritten:

◦ Wiederholung der folgenden Schritte:

▪ Bestimmung einer aktuellen transkraniellen Übertragung,
▪ Bestimmung (S30), dass die aktuelle transkranielle Übertragung kompatibel ist, wenn eine aktuelle Intensität vorliegt, z. B., wenn die Maskierungsintensität gleich der aktuellen Intensität ist und die transkranielle Übertragung des Patienten (200) gleich der aktuellen transkraniellen Übertragung ist; dann wird der Testklang vom kontralateralen Ohr (220) nicht wahrgenommen, und der Maskierungsklang verhindert nicht, dass das getestete Ohr (210) den Testklang wahrnimmt.

◦ Bestimmung der Maskierungsintensität aus einer während des Wiederholungsschritts bestimmten maximalen kompatiblen aktuellen transkraniellen Übertragung.

3. Audiometrisches Testverfahren nach dem vorhergehenden Anspruch, wobei das audiometrische Testverfahren die erste Dublette von Schritten umfasst und wobei die Bestimmung eines aktuellen Rinne-Testergebnisses einen Schritt zum Dekrement (S40) des aktuellen Rinne-Testergebnisses um einen Schritt umfasst; wobei das Verfahren ferner einen Schritt zur Initialisierung (S10) des aktuellen Rinne-Testergebnisses auf ein Initialisierung-Rinne-Testergebnis umfasst.

4. Audiometrisches Testverfahren nach einem der Ansprüche 2 oder 3, wobei das audiometrische Testverfahren die erste Dublette von Schritten umfasst und wobei, wenn das maximale kompatible aktuelle Rinne-Testergebnis unter einem Mindestwert liegt, eine Alarmmeldung gesendet wird.

5. Audiometrisches Testverfahren nach einem der Ansprüche 2 bis 4, wobei das audiometrische Testverfahren die erste Dublette von Schritten umfasst und wobei der Schritt zur Bestimmung, dass das aktuelle Rinne-Testergebnis kompatibel ist, folgende Schritte umfasst:

- Berechnung aus der Testintensität eines Wirksamkeitsschwellenwerts für die Maskierungsintensität, über dem der Testklang vom kontralateralen Ohr (220) nicht wahrgenommen werden kann;
- Berechnung aus der Testintensität eines Resonanzschwellenwerts für die Maskierungsintensität, unterhalb dessen der Maskierungsklang die Wahrnehmung des Testklangs durch das getestete Ohr (210) nicht verhindert;
- Schritt zum Vergleich des Wirksamkeitsschwellenwerts und des Resonanzschwellenwerts;

wobei das aktuelle Rinne-Testergebnis kompatibel ist, wenn der Wirksamkeitsschwellenwert kleiner oder gleich dem Resonanzschwellenwert ist.

6. Audiometrisches Testverfahren nach Anspruch 2 bis 5, wobei der audiometrische Test in Luftleitung durchgeführt wird, wobei der audiometrische Test die erste Dublette von Schritten umfasst und wobei die Berechnung des Wirksamkeitsschwellenwerts aus dem aktuellen Rinne-Testergebnis erfolgt.

7. Audiometrisches Testverfahren nach Anspruch 2 oder 6, wobei das audiometrische Testverfahren die erste Dublette von Schritten umfasst und wobei die Berechnung des Resonanzschwellenwerts aus dem aktuellen Rinne-Testergebnis erfolgt.

8. Audiometrisches Testverfahren nach einem der Ansprüche 5 bis 7, wobei das audiometrische Testverfahren die erste Dublette von Schritten umfasst und wobei im Schritt zur Bestimmung der Maskierungsintensität das aktuelle Rinne-Testergebnis als Wert das maximale kompatible aktuelle Rinne-Testergebnis annimmt:

   - wenn der Wirksamkeitsschwellenwert und der Resonanzschwellenwert gleich sind, nimmt die Maskierungsintensität als Wert den Wirksamkeitsschwellenwert an, oder
   - wenn der Wirksamkeitsschwellenwert kleiner als der Resonanzschwellenwert ist, nimmt die Maskierungsintensität einen Zwischenwert zwischen Wirksamkeitsschwellenwert und Resonanzschwellenwert als Wert an.

9. Audiometrisches Testverfahren nach einem der Ansprüche 2 bis 4, wobei das audiometrische Testverfahren die zweite Dublette von Schritten umfasst und wobei der Schritt zur Bestimmung, dass das aktuelle Rinne-Testergebnis kompatibel ist, die folgenden Schritte umfasst:

   - Berechnung aus der Testintensität einer ausreichenden Intensität, um den Testklang im kontralateralen Ohr (220) zu maskieren,
   - Berechnung der Resonanz des Maskierungsklangs im getesteten Ohr (210) aus ausreichender Intensität,

   wobei das aktuelle Rinne-Testergebnis kompatibel ist, wenn die Resonanz des Maskierungsklangs kleiner als die Testintensität plus ein Signal-Rausch-Verhältnis ist.

10. Audiometrisches Testverfahren nach einem der Ansprüche 1 bis 9, wobei:

   - der audiometrische Test in Luftleitung durchgeführt wird und das getestete Ohr (210) das bessere Ohr ist, das Testverfahren ferner einen audiometrischen Test des dem getesteten Ohr (210) gegenüberliegenden Ohrs (220) in Luftleitung umfasst, wobei
   - der audiometrische Test in Knochenleitung durchgeführt wird, und das getestete Ohr (210) das Ohr ist, bei dem ein Weber-Test lateralisiert wurde, wobei das Testverfahren ferner einen audiometrischen Test des dem getesteten Ohr (210) gegenüberliegenden Ohrs (220) in Knochenleitung umfasst.

11. Elektronische, audiometrische Testvorrichtung (100) zur Durchführung der Verfahrensschritte nach einem der Ansprüche 1 bis 10.

12. Computerprogramm umfassend Anweisungen, die die Vorrichtung nach Anspruch 11 dazu veranlassen, die Verfahrensschritte nach einem der Ansprüche 1 bis 10 durchzuführen.


**Claims**

1. Audiometry test method comprising the following steps:

   - Simultaneous application (S50) of a test sound to a test ear (210) of a patient (200) comprising a test intensity, and of a masking sound, comprising a masking intensity, to a contralateral ear (220) to mask the test sound,

   the process being **characterized in that** it comprises at least one of the following two pairs of steps:

   - The first pair of following steps:

- Determining a theoretical Rinne from a transcranial transfer,
- Determining masking intensity from theoretical Rinne

    - Or, the second pair of steps below:

- Determining a theoretical transcranial transfer from a theoretical Rinne,
- Determining a masking intensity from a theoretical transcranial transfer.

2. Audiometry test method according to the preceding claim further comprising at least one of the following two double steps, the patient (200) comprising a transcranial transfer:

    - First double steps:

    - Repeat the following steps:

- Determining a current Rinne,
- Determining (S30) that the current Rinne is compatible, if there is a current intensity, such that if the masking intensity is equal to the current intensity and the Rinne of the test ear (210) or contralateral ear (220) is equal to the current Rinne, then the test sound is not perceived by the contralateral ear (220) and the masking sound does not prevent perception of the test sound by the test ear (210),

- Determination of a masking intensity from a maximum compatible current Rinne determined during the repetition step,

    - Second double steps:

    - Repeat the following steps:

- Determining a current transcranial transfer,
- Determining (S30) that the current transcranial transfer is compatible, if there is a current intensity, such that if the masking intensity is equal to the current intensity and the patient's transcranial transfer (200) is equal to the current transcranial transfer, then the test sound is not perceived by the contralateral ear (220) and the masking sound does not prevent perception of the test sound by the tested ear (210),

- Determination of a masking intensity from a maximum compatible current transcranial transfer determined during the repetition step.

3. An audiometry test method according to the preceding claim, wherein the audiometry test method comprises the first double steps, and wherein the step of determining a current Rinne comprises a step of decrementing (S40) the current Rinne by one step, the method further comprising a step of initializing (S10) the current Rinne to an initialization Rinne.

4. An audiometry test method according to any one of claims 2 or 3 wherein the audiometry test method comprises the first double steps, and wherein if the maximum compatible current Rinne is below a minimum threshold, an alarm message is sent.

5. An audiometry test method according to any one of claims 2 to 4 wherein the audiometry test method comprises the first double steps, and wherein the step of determining that the current Rinne is compatible comprises the following steps:

- Calculation of an efficacy threshold for the masking intensity above which the test sound cannot be perceived by the contralateral ear (220), based on the test intensity,
- Calculation of a no-overmasking threshold for the masking intensity below which the masking sound does not prevent perception of the test sound by the ear under test (210), based on the test intensity,
- A step to compare the efficacy threshold and the no-overmasking threshold, the current Rinne being compatible if the efficacy threshold is less than or equal to the no-overmasking threshold.

6. Audiometry test method according to claims 2 to 5, the audiometry test being performed in air-conduction, wherein the audiometry test method comprises the first double steps, and wherein the calculation of the efficacy threshold is performed from the current Rinne.

7. Audiometry test method according to claim 2 or 6, wherein the audiometry test method comprises the first double steps, and wherein the threshold calculation is performed on the basis of the current Rinne.

8. An audiometry test method according to any one of claims 5 to 7 wherein the audiometry test method comprises the first double steps, and wherein, during the step of determining the masking intensity, the current Rinne taking as its value the maximum compatible current Rinne:

- If the efficacy threshold and the no-overmasking threshold are equal, then the masking intensity takes the efficacy threshold as its value, or
- If the efficacy threshold is lower than the no-overmasking threshold, then the masking intensity takes on an intermediate value between the efficacy threshold and the no-overmasking threshold.

9. An audiometry test method according to any one of claims 2 to 4 wherein the audiometry test method comprises the second double steps, and wherein the step of determining that the current Rinne is compatible comprises the following steps:

- Calculate an intensity sufficient to mask the test sound in the contralateral ear (220) from the test intensity,
- Calculation of the impact of the masking sound in the test ear (210) based on the intensity,

the current Rinne being compatible if the masking sound is less than the test intensity plus a signal-to-noise ratio.

10. Audiometry test method according to any one of claims 1 to 9 in which:

- The audiometry test is performed in air-conduction, and the tested ear (210) is the better ear, the test method then comprising an audiometry test of the opposite ear (220) to the tested ear (210) in air-conduction, where
- The audiometry test is carried out in bone-conduction, and the tested ear (210) is the ear towards which a Weber test was lateralized, the test method then comprising an audiometry test of the opposite ear (220) to the tested ear (210) in bone-conduction.

11. Electronic audiometry testing device (100) configured to implement the steps of the process according to any one of claims 1 to 10.

12. Computer program comprising instructions that cause the device according to claim 11 to perform the steps of the process according to any one of claims 1 to 10.

[Fig. 1]

100

110

120

130

210

220

200

[Fig. 2]

S10

S20

S30

S40

S50

[Fig. 3]

# RÉFÉRENCES CITÉES DANS LA DESCRIPTION

## Documents brevets cités dans la description

- US 2004097826 A1

- JP S56139740 A

## Littérature non-brevet citée dans la description

- **WHITTLE et al.** A determination of the normal threshold of hearing by bone conduction. *JOURNAL OF SOUND AND VIBRATION*, July 1965, vol. 2 (3), ISSN 0022-460X
- **DOBIE R A et al.** Binaural interaction in auditory brain-stem responses: effects of masking. *ELECTROENCEPHALOGRAPHY AND CLINICAL NEUROPHYSIOLOGY/EVOKED POTENTIALS SECTION*, January 1985, vol. 62 (1), ISSN 0168-5597
- **MARKLE O M et al.** The audiometer weber test as a means of determining the need for, and type of, masking. *ANN. OTOL., ETC. 1952*, 1952, vol. 61 (3), 888-900

- Clinical Masking. **KATZ, J.** ; **LEZYNSKI, J.** Handbook of Clinical Audiology. Williams and Wilkins, 2002
- **MUNRO, K.J** ; **AGNEW, N**. A comparison of interaural attenuation with the Etymotic ER-3A insert earphone and the Telephonics TDH-39 supra-aural earphone. *Br J Audiol*, 1999, vol. 33, 259-262
- **HALL, JW.** ; **MUELLER, HG.** The audiologists' desk reference. Singular Publishing Group, 1997, vol. I
- **MUNRO** ; **AGNEW**. *BJA*, 1999
- **HALL J.W. III** ; **MUELLER G.H. III / MUNRO** ; **AGNEW**. *BJA*, 1999
- **KATZ J** ; **MUNRO** ; **AGNEW**. *BJA*, 1999
- **HALL J.W. III** ; **MUELLER G.H. III** ; **MUNRO** ; **AGNEW**. *BJA*, 1999